# EUROPEAN PATENT SPECIFICATION

(11) **EP 1 961 261 B1**
(45) Date of publication and mention of the grant of the patent: **13.03.2013**
(21) Application number: 06841041.4
(22) Date of filing: 15.12.2006
(51) Int. Cl.: H04L 25/02, H04W 52/02

(54) **CONTROL CIRCUITRY FOR PROVIDING AN INTERFACE BETWEEN CONNECTABLE TERMINAL AND PERIPHERAL DEVICE CIRCUITRY**
STEUERSCHALTUNG ZUR HERSTELLUNG EINER SCHNITTSTELLE ZWISCHEN EINEM ANSCHLIESSBAREN ENDGERÄT UND EINER PERIPHERGERÄTESCHALTUNG
CIRCUITS DE COMMANDE PERMETTANT DE FOURNIR UNE INTERFACE ENTRE DES CIRCUITS DE DISPOSITIFS TERMINAUX ET PÉRIPHÉRIQUES CONNECTABLES

(30) Priority: 16.12.2005 GB 0525632
(43) Date of publication of application: 27.08.2008
(73) Proprietor: Nokia Corporation, 02150 Espoo (FI)
(72) Inventor: LINDLAR, Heribert, 44795 Bochum (DE); SATTLER, Stefan, D-80339 Munich (DE)
(74) Representative: Khan, Mohammed Saiful Azam
(86) International application number: PCT/EP2006/012249
(87) International publication number: WO 2007/068500

(56) References cited:
- EP-A- 1 484 664
- US-A- 5 686 813
- US-A1- 2001 004 198
- US-A1- 2003 101 303
- US-A1- 2005 242 975

## Description

The invention relates to control circuitry for providing an interface between connectable terminal and peripheral device circuitry.

### Background

Numerous accessories or peripheral devices can be connected to electronic devices, such as mobile phone handsets. The device to which the peripheral device or accessory is connected is called a terminal. An example of an accessory that can be connected to various terminals is a headset. An interface between a terminal and an accessory can be implemented either using a wire connector or alternatively a wireless connection. On a single wire connection, it may be necessary to transfer audio and data signals and the terminal may also supply power to the accessory.

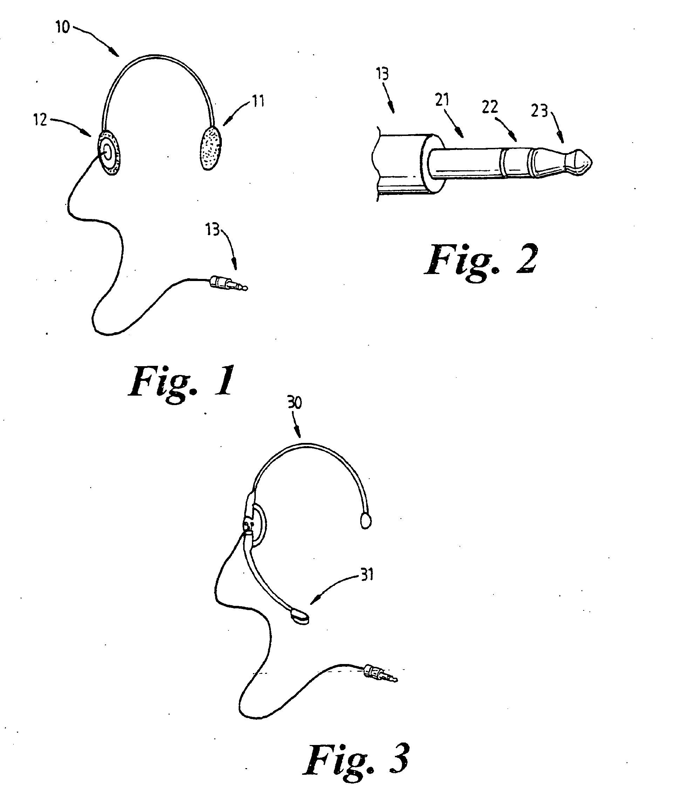

Figure 1 presents a headphone 10 that can be connected, for instance, to a mobile phone handset. The headphone 10 includes a left speaker 11, a right speaker 12 and a plug 13. Figure 2 presents a plug 13 of the headphone 10 of Figure 1 in more detail. The body of the plug 13 includes a sleeve 21, a ring 22 and a terminating tip 23, each providing contact points with a jack in the terminal. These plug contacts are often referred to as poles. In this case the tip 23 is connected to the left speaker 11, the ring 22 is connected to the right speaker 12, and the plug sleeve 21 serves as a ground connection.

Standardised audio/video (A/V) plugs and jacks are frequently used in consumer audio and telecommunication products. A/V plugs are familiar to most people, with a typical A/V comprising a series of electrically isolated cylindrical segments ending in a. tip_segment..

Figure 3 presents a headset 30. In this headset 30, in addition to left and right speakers and a plug, there is a microphone 31. Therefore, the plug portion of this headset may comprise four contacts points: one sleeve, two rings and a tip. The extra ring is used for the microphone. Alternatively, a plug with three contacts can be used, but in this case the same signals are led to the left and right speakers.

There exist also more advanced headset-terminal configurations, in which some control signals are transferred between the terminal and the headset.

These control signals can be, for instance, volume adjustment signals, signals for controlling the call (on hook or off hook) or signals for controlling the operation of a music player.

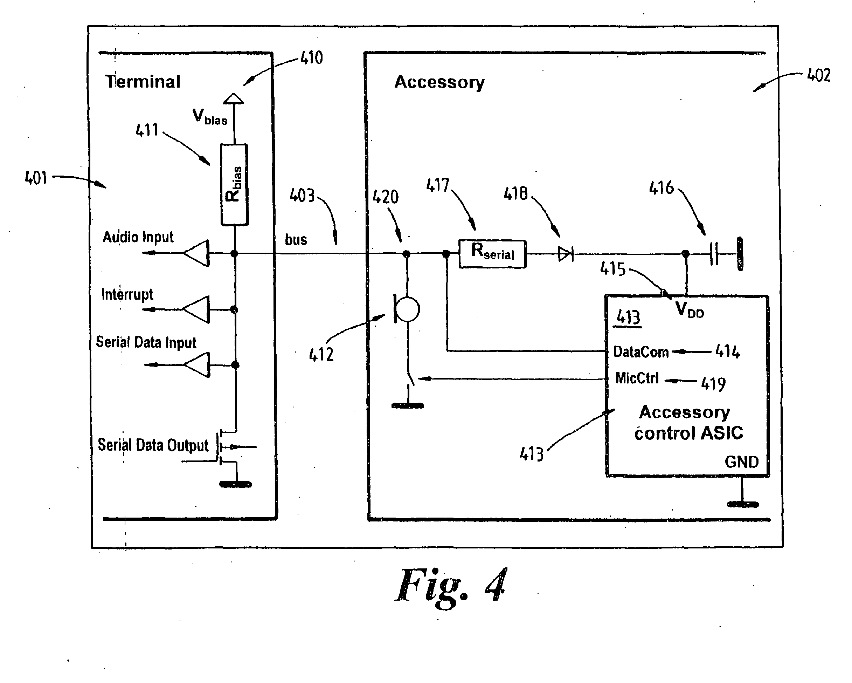

Figure 4 shows a block diagram of such an advanced headset-terminal configuration. The system consists of a terminal 401, an accessory 402 and a single wire bus 403 between them. In this text the bus is the connector between the terminal 401 and an application specific integrated circuit (ASIC) 413 of the accessory 402. The system in this prior art solution works so that on the terminal 401 side there is a bias supply V_{bias} 410 of 2 to 2.5 V connected via a resistor, R_{bias} 411 (usually 2.2k ohms) to the single wire bus. On the accessory 402 side a microphone 412, for instance a condenser microphone, is connected to the bus 403. The resistor R_{bias} 411 translates the modulated microphone current to an alternating current (AC) voltage amplified further in the audio stages of the terminal 401. On the accessory 402 side, a MicCtrl 419 is used to control the operation of the microphone 412.

For accessory control and signalling any user interaction to the terminal 401, the low-power ASIC 413 is connected to the bus 403 too. It also uses R_{bias} 411 as a working resistor for digital signaling with "open-drain" type outputs on the terminal 401 and the accessory 402 side. The "open-drain" output is an output signal where pulling low (0 bit) is done by the FET of an ASIC whereas pulling high (1 bit) is done by an external resistor. The DataCom pin 414 serves as an input/output (I/O) on the accessory 402 side.

The ASIC 413 also has to receive its supply voltage via the bus 403. When connecting a V_{DD} 41 5 of the accessory ASIC 413 directly to the bus 403, its supply capacitor 416 becomes a short-circuit to audio and data signals since it has usually quite a large capacitance (e.g. 47 µF) and has a low impedance at the audio frequencies. Therefore, a resistor Rₛₑᵣᵢₐₗ 417 is needed to decouple the terminal 401 from the V_{DD} 415. The value of the Rₛₑᵣᵢₐₗ 417 must not be too large since too resistive Rₛₑᵣᵢₐₗ 417 would result in too low V_{DD} supply voltage. Current technologies require V_{DD} to be 1 to 1.5 V at least. When digital signaling takes place on the bus, logic-low data (0 bit) means a voltage from node 420 to GND close to 0 V. During logic low pulses (0 bits), the supply capacitor 416 would be discharged via Rₛₑᵣᵢₐₗ quite fast without a diode 418. To prevent this the diode 418 is used between the V_{DD} 415 and the Rₛₑᵣᵢₐₗ 417 to avoid any current flowing back from the ASIC's 413 supply capacitor 416. Although the positions of the Rₛₑᵣᵢₐₗ 417 and the diode 418 could be interchanged this would make it problematic to integrate the diode 418 with the ASIC 413.

Unfortunately the non-linear characteristic of the diode 418 has a rectification effect on the audio signals on the bus 403. With reasonable values of Rₛₑᵣᵢₐₗ (800 ohms to 2k ohms) this may lead to unacceptable distortions in the audio signal.

The varying impedance of the diode 418 is also a problem. The impedance of the diode 418 is in the some range (500 ohms) as a reasonable Rₛₑᵣᵢₐₗ and it varies with the ASIC's 413 supply current, which varies with temperature, and operating state (activity). The impedance of the diode 418 also varies from one component to another. The diode 418 and Rₛₑᵣᵢₐₗ 417 form an impedance, which is parallel to R_{bias} 411. The microphone 412 AC current is converted to a microphone 412 voltage at the terminal 401 input using a factor determined by the above mentioned varying parallel impedance. This leads to an undesired varying audio level.

US2005/242975 describes an integrated speaker or microphone which derives its power from an incoming signal, thereby minimizing or eliminating the need for an external amplifier or power supply. A system according to the invention includes an audio transducer, an input for receiving a digital audio signal, an amplifier for amplifying the digital audio signal to or from the audio transducer, and a rectifier for rectifying the digital audio signal for use in powering the amplifier. A filter is included for filtering the output of the rectifier. In the preferred embodiment, the digital audio signal uses a non-return-to-zero (NRZ) encoding scheme. When the transducer is a microphone, it is connected to an analog-to-digital (A/D) converter, also powered by the rectified digital audio signal.

### Summary

Described herein is povided a control circuitry as set furth is claim 1, and a method as set furth is claim 107.

Described herein there is provided control circuitry for providing an interface between connectable terminal and peripheral device circuitry, wherein the peripheral device circuitry comprises
a bus line for transferring data and power between the peripheral device circuity and the terminal device circuitry;
a charge storage device arranged to receive power from the terminal device circuitry over the bus line and to supply the power to the control circuitry;
wherein the control circuitry is operable to connect the charge storage device to the bus line to receive power for storage in response to the terminal device circuitry transmitting a first data value represented by a higher voltage level and to disconnect the charge storage device from the bus line in response to the terminal device circuitry transmitting a second data value represented by a lower voltage level to prevent the charge storage device from discharging over the bus.

In this way, it is possible to transfer data (including audio signals) and power to the peripheral device via the interface, and the audio performance of the peripheral device is greatly improved. The control circuitry is cheap to produce and consumes very little space. In terms of price and size, the control circuitry is comparable to the diode solution. The control circuitry can easily be integrated into an ASIC.

The control circuitry may be operable to disconnect the charge storage device from the bus line in response to the peripheral device circuitry transmitting the data value represented by the lower voltage level.

The control circuitry may include a switch for effecting the connection and disconnection of the charge storage device and bus line.

The switch may be a p-channel enhancement mode metal oxide semiconductor field effect transistor.

Also described herein there is provided an application specific integrated circuit (ASIC) comprising the control circuitry of the first aspect.

Also described herein is provided peripheral device circuitry comprising control circuitry also described herein

Also described herein there is provided a system comprising at least terminal device circuitry and peripheral device cirruitry also described herein

Also described herein there is provided peripheral device circuitry for providing an interface between connectable terminal and peripheral device circuitry, wherein the peripheral device circuitry comprises
a bus line for transferring data and power between the peripheral device circuitry and the terminal device circuitry;
control circuitry;
a charge storage device arranged to receive power from the terminal device circuitry over the bus line and to supply the power to the control circuitry;
wherein the control circuitry is operable to connect the charge storage device to the bus line to receive power tor storage :n response to the terminal device circuitry transmitting a first data value represented by a higher voltage level and to disconnect the charge storage device from the bus line in response to the terminal device circuitry transmitting a second data value represented by a lower voltage level, to prevent the charge storage device from discharging over the bus.

Also described herein there is provided peripheral device comprising control circuitry also described herein.

Also described herein there is provided a peripheral device comprising peripheral device circuitry also described herein.

Also described herein there is provided means for controlling an interface between connectable terminal and peripheral device circuitry, wherein the peripheral device circuitry comprises
means for transferring data and power between the peripheral device circuitry and the terminal device circuitry;
means for receiving power from the terminal device circuitry over the means for transferring data and power, and for supplying the power to the control circuitry;
wherein the means for controlling is operable to connect the means for receiving and supplying power to the means for transferring data and power to receive power for storage in response to the terminal device circuitry transmitting a first data value represented by a higher voltage level, and to disconnect the means for receiving and supplying power from the means for transferring data and power in response to the terminal device circuitry transmitting a second data value represented by a lower voltage level, to prevent the means for receiving and supplying power from discharging over the means for transferring data and power.

Also described herein there is provided a method of providing an interface between connectable terminal and peripheral device circuitry, the method comprising
connecting a charge storage device to a bus line to receive power for storage in response to the terminal device circuitry transmitting a first data value represented by a higher voltage level, and disconnecting the charge storage device from the bus line in response to the terminal device circuitry transmitting a second data value represented by a lower voltage level, to prevent the charge storage device from discharging over the bus.

The method may comprise disconnecting the charge storage device from the bus line in response to the peripheral device circuitry transmitting the data value represented by the lower voltage level.

Also described herein there is provided a method of providing an interface betwen connectable terminal and peripherar device circuitry, the method comprising
a step for connecting a charge storage device to a bus line to receive power for storage in response to the terminal device circuitry transmitting a first data value represented by a higher voltage level, and a step for disconnecting the charge storage device from the bus line in response to the terminal device circuitry transmitting a second data value represented by a lower voltage level, to prevent the charge storage device from discharging over the bus.

Also described herein there is provided a computer-readable medium having computer-executable components for providing an interface between connectable terminal and peripheral device circuitry, comprising
a component for connecting a charge storage device to a bus line to receive power for storage in response to the terminal device circuitry transmitting a first data value represented by a higher voltage level, and a component for disconnecting the charge storage device from the bus line in response to the terminal device circuitry transmitting a second data value represented by a lower voltage level, to prevent the charge storage device from discharging over the bus.

Also described herein there is provided a computer program comprising program code means adapted to perform any of the steps of methods described herein when the program is run on a processor.

Also described herein there is provided a computer program product comprising program code means stored in a computer-readable medium, the program code means being adapted to perform any of the steps of the method described herein when the program is run on a processor.

Any circuitry may include one or more processors, memories and bus lines. One or more of the circuitries described may share circuitry elements.

The present disclosure includes one or more aspects, embodiments or features in isolation or in various combinations whether or not specifically stated (including claimed) in that combination or in isolation.

The above summary is intended to be merely exemplary and non-limiting.

### Brief Description Of The Figures

These and other features of the present invention will by way of example become apparent from the following detailed description when considered in conjunction with the accompanying drawings, in which:
Figure 1 illustrates a headphone;
Figure 2 shows an A/V plug;
Figure 3 illustrates a headset;
Figure 4 is a schematic block diagram of a prior art headset-terminal configuration;
Figure 5 is a schematic block diagram of a headset-terminal configuration;
Figure 6 is a schematic block diagram of a headset-terminal configuration;
Figure 7 represents output characteristics of a p-channel transistor.
Figure 8 represents output characteristics of a p-channel transistor showing all four quadrants of the I_{D} versus V_{ds} graph;
Figure 9 is a flowchart representing a method of providing an interface between connectable terminal and peripheral device circuitry.

### Detailed Description

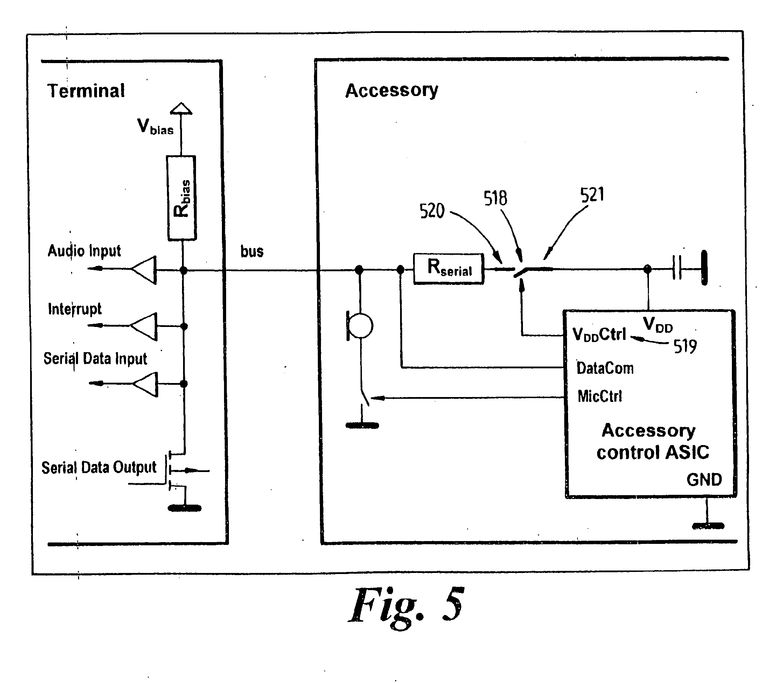

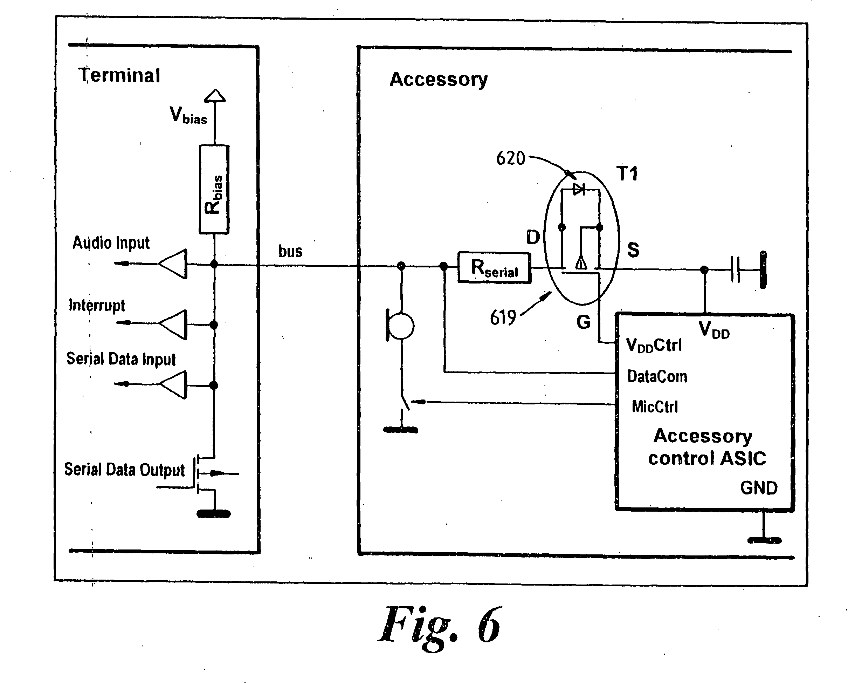

Figs. 5 and 6 are identical to Figure 4, apart from the diode 418, which is now replaced with a switch and there is also a new pin on the accessory side 413; V_{DD}Ctrl 519 for controlling the switch. Like numerals are used to describe the same blocks throughout this description. The interface between the terminal 401 and the ASIC 413 of the accessory 402 is in this description called a bus, which can be, for instance, a wire or connector between the terminal 401 and the ASIC 413 of the accessory 402.

In Figure 5 the diode 418 of Figure 4 is replaced with a switch 518, in this case an analogue switch. The switch 518 can be controlled by the V_{DD}Ctrl 519 on the accessory side. The switch can either be open (highly resistive) or closed (very little resistive). One end (node 520) of the switch 518 is connected to the Rₛₑᵣᵢₐₗ 417 and the other end (node 521) is connected to V_{DD} 415 of the ASIC 413 of the accessory 402. The end (node 520) that is connected to the Rₛₑᵣᵢₐₗ 417 is predominantly (always, except if the terminal 401 or the accessory 402 pulls logic low (0 bit) on the bus) substantially at the same voltage than the end (node 521) that is connected to the V_{DD} 415 since the switch 518 is closed. In this text when referred to voltages, they are measured to ground unless otherwise mentioned. The switch 518 is a low impedance switch controlled by the accessory ASIC 413. When the bus 403 is pulled logic high by R_{bias} 411, the switch 518 should be closed to supply power to the ASIC 413. Logic high is the default value on the bus 403.

The switch 518 is predominantly (always except when a logic low is pulled on the bus 403) closed (low resistance) and allows the ASIC 413 to be supplied via the Rₛₑᵣᵢₐₗ 417. Since the resistance of the switch 518 is in the range of few ohms with a very low voltage drop across it, the inevitable forward voltage drop of 0.2 V to 0.5 V of the diode 418 of Figure 4 can be consumed by the Rₛₑᵣᵢₐₗ 417. Therefore, the resistance value of the Rₛₑᵣᵢₐₗ 417 can be higher than in the prior art solutions. The Rₛₑᵣᵢₐₗ 417 can now be in the range of 2k to 4k ohms (in current solutions as described with reference to Figure 4, the Rₛₑᵣᵢₐₗ 417 cannot be much higher than 1k ohm). This means that Rₛₑᵣᵢₐₗ 417 can now dominate the resistance of the switch 518 and any possible non-linearity of the resistance of the switch 518. The microphone current is translated to an AC voltage by the parallel resistance of the R_{bias} 411 and Rₛₑᵣᵢₐₗ 417. Since now the Rₛₑᵣᵢₐₗ 417 is higher, more usable AC voltage is available at the terminal 401 input. The dominance of the Rₛₑᵣᵢₐₗ 417 with regard to the switch 518 resistance also minimises any effect of temperature variations on the AC input voltage at the terminal 401 due to the resistance of the switch 518.

The ASIC 413 provides control to the switch 518 so that it is open (high resistive) all times, when a logic low (0 bit) is pulled on the bus 403 by the terminal 401 or the accessory 402 since the supply capacitor 416 could discharge through Rₛₑᵣᵢₐₗ 417 when logic low is pulled on the bus 403. As a result the supply capacitor 416 of the ASIC 413 is disconnected from the bus when current could flow back towards the terminal 401. The ASIC 413 knows itself when it pulls the bus a logic low because it signals information to the terminal 401. Furthermore, it can observe the bus 403 via a bi-directional DataCom pin 414 and can also open the switch 518 by using V_{DD}Ctrl pin, when the terminal 401 is sending a logic low to the accessory 402. Every time when logic one is pulled on the bus 403, the switch 518 should be quickly closed to allow the supply capacitor 416 to be charged.

In Figure 6, a p-channel enhancement mode metal oxide semiconductor field effect transistor (P-MOSFET) 619 operates as a switch. As can be seen from Figure 6, gate of the transistor 619 is connected to the V_{DD}Ctrl 719, source is connected to the V_{DD} 415 of the ASIC 413 and drain is connected to the Rₛₑᵣᵢₐₗ 417. The transistor 619 can now be controlled by the V_{DD}Ctrl 519 so that the transistor 619 operates as specified in its data sheets (gate voltage negative to source to make the transistor conductive from drain to source).

Logic high voltage level at the V_{DD}Ctrl 1519 (close to V_{DD}) results in a gate-to-source voltage V_{gs} close to 0 V, which sets the transistor 619 to a very high impedance between the drain and the source (switch open). Logic low level at the V_{DD}Ctrl 519 (close to GND) applies a voltage of about V_{DD} across gate-source with a negative gate voltage with respect to the source voltage. The supply voltage V_{DD} and thus |V_{gs}| is larger than 1 V since that is the minimum operational voltage even for a low-power ASIC.

The transistor 619 has a low threshold voltage of 0.4 V to 0.8 V and has a low on-resistance. When the gate voltage is more negative than the threshold with regard to the source (here the V_{DD} node), the drain-to-source channel becomes very little resistive in the range of a few ohms (switch closed, i.e. transistor conductive).

Normally when P-MOSFET transistors are applied to other applications, the drain voltage is negative with regard to the source (V_{ds}) and if the gate controls the MOSFET to be conductive, then the current is flowing from the source to the drain. However, the drain voltage is predominantly positive with regard to the source (V_{ds}), which is usually not explicitly specified in P-MOSFET's datasheets. V_{ds} is negative only when a logic low is pulled on the bus 403. If the P-MOSFET 619 was still controlled to be low-resistive, it would allow current flow from the source to the drain and thus discharging the supply capacitor 416. However, when logic low is pulled, the MOSFET 619 needs to be controlled by the ASIC 413 so that it is highly resistive.

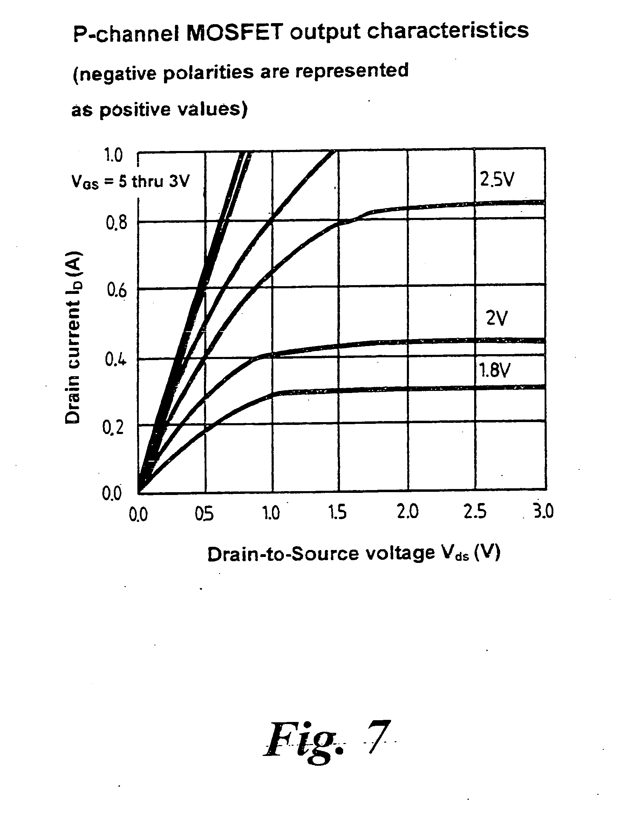

Figure 7 describes output characteristics of a typical p-channel MOSFET as usually published by transistor vendors. On the horizontal axis there is the negative drain-to-source voltage, V_{ds} and on the vertical axis there is the negative drain current, I_{D}. Each curve in the figure represents output characteristics with different negative V_{gs} values. Figure 7 only shows the first quadrant of the graph (other three quadrants are not visible in this figure).

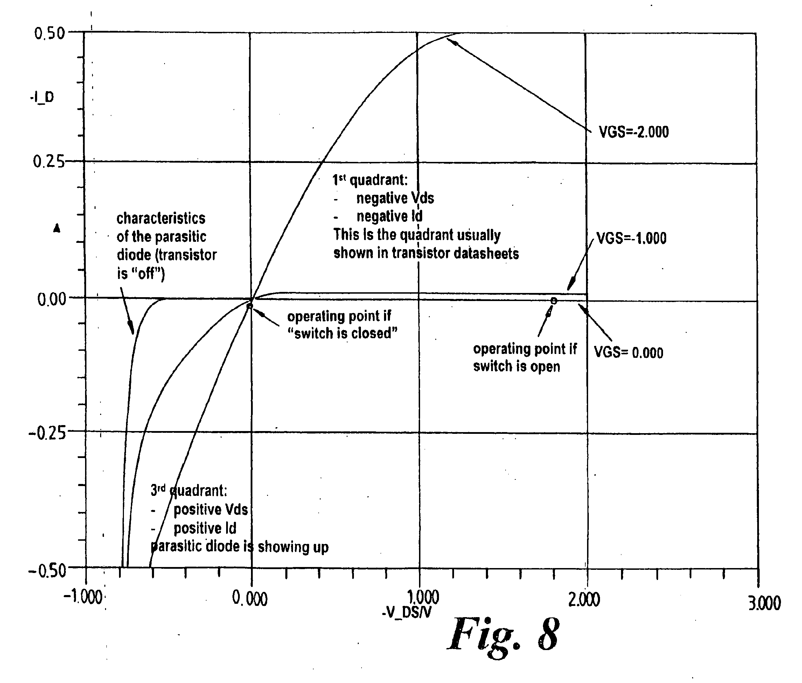

Figure 8 shows all 4 quadrants of the -I_{D} versus -V_{ds} graph. The transistor 619 predominantly (when the switch is closed) operates in the lower left corner (third quadrant) of this -I_{D} versus -V_{ds} graph, close to the origin with positive I_{D} (e.g. +50 µA) and V_{ds} (e.g. +10 mV) not visible in Figure 7. This is the case, when the ASIC 413 controls the gate so that the switch is closed (i.e. the transistor is low-resistive). V_{gs} can then be about -1.8 V.

When the V_{gs} equals to -2 V, it can be seen from Figure 8 that the curve corresponding to V_{gs} = -2 V is almost a straight line in the operating point close to the origin. This is an additional reason, why this transistor switch solution provides extremely low audio distortion compared to the diode solution in Figure 4.

The transistor 619 operates in the first quadrant (upper right) of the graph of Figure 8 for short periods only, when the switch is open (transistor highly resistive), because a logic low is pulled on the bus 403. Exemplary values can be for instance: bus voltage ≈ 0-V, V_{DD} ≈ 1.8 V, V_{ds} ≈ -1.8 V, V_{gs} ≈ 0 V and I_{D} ≈ I nA. In Figure 8 this operational point is on the V_{ds} axis at 1.8 V.

P-channel MOSFET transistors have a parasitic diode 620 as shown in Figure 6. In usual applications according to the FET data sheet, it is reverse biased (cathode positive with regard to anode) and thus not conducting. However, the parasitic diode 620 helps in the start-up phase of the accessory 402, when V_{DD} 415 is initially 0 V. Without the parasitic diode 620, the ASIC 413 would not be able to apply any voltage to the gate to make the transistor 619 conductive. As a result V_{DD} 41 5 would not start rising, and there would be no current flow from the drain to the source - a deadlock. The parasitic diode 620 however is conducting and supplies current to the supply capacitor 416 and the V_{DD} 415 of the accessory 413 will ramp up. As soon as the V_{DD} 415 is somewhat larger than the threshold of the transistor 619, the current is no longer flowing through the parasitic diode, but the transistor 619 becomes conductive and finally has the resistance of only few ohms. The parasitic diode 620 is no longer visible, for instance to the audio signals, since it is shorted by the conducting transistor.

Instead of using the p-channel MOSFET 619, a bipolar transistor may be applied as well. However, bipolar transistors do not provide the parasitic diode and therefore an extra component could be used to replace the parasitic diode 620.

The switch 18, 619 may be connected to the bus 403.

The switch 18, 619 can be physically located in the accessory 402.

A system may comprise at least the switch 518, 619, the accessory 402, the bus 403 and the terminal 401.

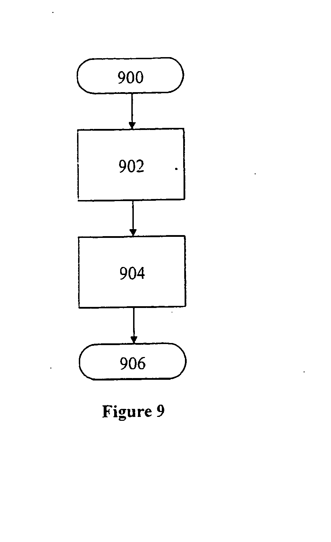

Figure 9 is a flowchart representing a method of providing an interface between connectable terminal and peripheral device circuitry.

The-method begins ar 900, and includes (902) connecting a charge storage device to a bus line to receive power for storage in response to the terminal device circuitry transmitting a first data value represented by a higher voltage level, and (904) disconnecting the charge storage device from the bus line in response to the terminal device circuitry transmitting a second data value represented by a lower voltage level, to prevent the charge storage device from discharging over the bus. The method ends at 906.

It will be appreciated that the aforementioned circuitry may have other functions in addition to the mentioned functions, and that these functions may be performed by the same circuit.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description it will be evident to a person skilled in the art that various modifications may be made within the scope of the invention.

It is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

Furthermore, in the claims means-plus- function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. Control circuitry (518) of a peripheral device (402), configured to be operable to:
connect:
a bus line of peripheral device circuitry, the bus line being arranged to be able to transfer data and power between the peripheral device circuitry and connectable terminal device circuitry; to
a charge storage device (416) of the peripheral device circuitry, the charge storage device being arranged to be able to receive power from the terminal device circuitry over the bus line and to supply power to the control circuitry (518), in response to the connectable terminal device circuitry transmitting a first data value represented by a higher voltage level to allow the charge storage device (416) to receive power for storage; and
disconnect the bus line from the charge storage device (416) in response to the terminal device circuitry transmitting a second data value represented by a lower voltage level to prevent the charge storage device (416) from discharging over the bus line.

2. The control circuitry (518) of any preceding claim being configured to be operable to disconnect the bus line from the charge storage device (416) in response to the peripheral device circuitry transmitting the data value represented by the lower voltage level.

3. The control circuitry (518) of any preceding claim including a switch for effecting the connection and disconnection of the charge storage device (416) and bus line.

4. The control circuitry (518) of claim 3, wherein the switch is a p-channel enhancement mode metal oxide semiconductor field effect transistor (619).

5. The control circuitry (518) of any preceding claim, wherein the charge storage device (416) is a capacitor.

6. An application specific integrated circuit (ASIC, 413) comprising the control circuitry (518) of any preceding claim.

7. Peripheral device circuitry comprising:
the control circuitry (518) of any of claims 1 to 5; or
the application specific integrated circuit (ASIC) (413) of claim 6.

8. A peripheral device comprising one of the following:
the peripheral device circuitry of claim 7;
the control circuitry (518) of any of claims 1 to 5; or
the application specific integrated circuit (ASIC) (413) of claim 6.

9. A system comprising:
at least terminal device circuitry; and
one of the peripheral device circuitry of claim 7 or the peripheral device of claim 8.

10. A method, comprising
connecting:
a bus line of peripheral device circuitry, the bus line being arranged to be able to transfer data and power between the peripheral device circuitry and connectable terminal device circuitry; to
a charge storage device (416) of the peripheral device circuitry, the charge storage device (416) being arranged to be able to receive power from the terminal device circuitry over the bus line and to supply power to control circuitry (518), in response to the terminal device circuitry transmitting a first data value represented by a higher voltage level to allow the charge storage device (416) to receive power for storage; and
disconnecting the bus line from the charge storage device (416) in response to the terminal device circuitry transmitting a second data value represented by a lower voltage level to prevent the charge storage device (416) from discharging over the bus line.

11. The method of claim 10 comprising disconnecting the charge storage device (416) from the bus line in response to the peripheral device circuitry transmitting the data value represented by the lower voltage level.

12. A computer program product comprising program code stated in a computer-readable medium, the computer program code being configured to perform any of the steps of any of claims 10 and 11 when the program is run on a processor.

## Patentansprüche

1. Steuerschaltung (518) eines Peripheriegeräts (402), konfiguriert, um betriebsfähig zu sein zum Verbinden einer Busleitung einer Schaltung eines Peripheriegeräts, wobei die Busleitung eingerichtet ist, um Daten und Strom zwischen der Schaltung eines Peripheriegeräts und einer Schaltung eines Endgeräts übertragen zu können, die verbunden werden kann, mit einer Ladungs-Speicherungsvorrichtung (416) der Schaltung des Peripheriegeräts, wobei die Ladungs-Speicherungsvorrichtung eingerichtet ist, Strom von der Schaltung des Endgeräts über die Busleitung empfangen zu können und der Steuerschaltung (518) Strom zuführen zu können, in Reaktion darauf, dass die Schaltung des Endgeräts, die verbunden werden kann, einen ersten Datenwert sendet, der durch ein höheres Spannungsniveau dargestellt wird, um es zu gestatten, dass die Ladungs-Speicherungsvorrichtung (416) Strom zum Speichern empfängt, und zum Trennen der Busleitung von der Ladungs-Speicherungsvorrichtung (416) in Reaktion darauf, dass die Schaltung des Endgeräts einen zweiten Datenwert sendet, der durch ein niedrigeres Spannungsniveau dargestellt wird, um zu verhindern, dass die Ladungs-Speicherungsvorrichtung (416) über die Busleitung entladen wird.

2. Steuerschaltung (518) gemäß einem vorstehenden Anspruch, konfiguriert, um betriebsfähig zu sein, die Busleitung von der Ladungs-Speicherungsvorrichtung (416) in Reaktion darauf zu trennen, dass die Schaltung des Peripheriegeräts den Datenwert sendet, der durch das niedrigere Spannungsniveau dargestellt wird.

3. Steuerschaltung (518) gemäß einem der vorstehenden Ansprüche, einschließend einen Schalter, um das Verbinden und Trennen der Ladungs-Speicherungsvorrichtung (416) und der Busleitung auszuführen.

4. Steuerschaltung (518) gemäß Anspruch 3, wobei der Schalter ein P-Kanal-MetallOxid-Halbleiter-Feldeffekttransistor (619) vom Anreicherungstyp ist.

5. Steuerschaltung (518) gemäß einem der vorstehenden Ansprüche, wobei die Ladungs-Speicherungsvorrichtung (416) ein Kondensator ist.

6. Anwendungsspezifische integrierte Schaltung (ASIC, 413) umfassend die Steuerschaltung (518) gemäß einem der vorstehenden Ansprüche.

7. Schaltung eines Peripheriegeräts, umfassend:
die Steuerschaltung (518) gemäß einem der Ansprüche 1 bis 5, oder
die anwendungsspezifische integrierte Schaltung (ASIC, 413) gemäß Anspruch 6.

8. Peripheriegerät, umfassend eines der folgenden:
Schaltung eines Peripheriegeräts, gemäß Anspruch 7,
die Steuerschaltung (518) gemäß einem der Ansprüche 1 bis 5, oder
die anwendungsspezifische integrierte Schaltung (ASIC, 413) gemäß Anspruch 6.

9. System, umfassend:
mindestens eine Schaltung eines Endgeräts, und
die Schaltung eines Peripheriegeräts gemäß Anspruch 7 oder das Peripheriegerät gemäß Anspruch 8.

10. Verfahren, umfassend:
Verbinden einer Busleitung einer Schaltung eines Peripheriegeräts, wobei die Busleitung eingerichtet ist, Daten und Strom zwischen der Schaltung eines Peripheriegeräts und einer Schaltung eines Endgeräts übertragen zu können, die verbunden werden kann, mit einer Ladungs-Speicherungsvorrichtung (416) der Schaltung des Peripheriegeräts, wobei die Ladungs-Speicherungsvorrichtung (416) eingerichtet ist, Strom von der Schaltung des Endgeräts über die Busleitung empfangen zu können und der Steuerschaltung (518) Strom zuführen zu können, in Reaktion darauf, dass die Schaltung des Endgeräts einen ersten Datenwert sendet, der durch ein höheres Spannungsniveau dargestellt wird, um es zu gestatten, dass die Ladungs-Speicherungsvorrichtung (416) Strom zum Speichern empfängt, und
Trennen der Busleitung von der Ladungs-Speicherungsvorrichtung (416) in Reaktion darauf, dass die Schaltung des Endgeräts einen zweiten Datenwert sendet, der durch ein niedrigeres Spannungsniveau dargestellt wird, um zu verhindern, dass die Ladungs-Speicherungsvorrichtung (416) über die Busleitung entladen wird.

11. Verfahren gemäß Anspruch 10, umfassend:
Trennen der Ladungs-Speicherungsvorrichtung (416) von der Busleitung in Reaktion darauf, dass die Schaltung des Peripheriegeräts den Datenwert sendet, der durch das niedrigere Spannungsniveau dargestellt wird.

12. Computerprogramm-Produkt, umfassend Programmcode, der in einem computerlesbaren Medium gespeichert ist, wobei der Computerprogrammcode konfiguriert ist, jeden der Schritte eines der Ansprüche 10 und 11 auszuführen, wenn das Programm auf einem Prozessor ausgeführt wird.

## Revendications

1. Montage de circuits de commande (518) d'un dispositif périphérique (402), configuré afin d'être exploitable de manière à :
connecter :
une ligne de bus de montage de circuits de dispositifs périphériques, la ligne de bus étant agencée de manière à être apte à transférer des données et de l'énergie entre le montage de circuits de dispositifs périphériques et le montage de circuits de dispositifs terminaux connectables ; à
un dispositif de stockage de charge (416) du montage de circuits de dispositifs périphériques, le dispositif de stockage de charge étant agencé de manière à être apte à recevoir de l'énergie provenant du montage de circuits de dispositifs terminaux sur la ligne de bus, et à fournir de l'énergie au montage de circuits de commande (518), en réponse à la transmission, par le montage de circuits de dispositifs terminaux connectables, d'une première valeur de données représentée par un niveau de tension supérieur, en vue de permettre au dispositif de stockage de charge (416) de recevoir de l'énergie pour un stockage ; et
déconnecter la ligne de bus du dispositif de stockage de charge (416) en réponse à la transmission, par le montage de circuits de dispositifs terminaux, d'une seconde valeur de données représentée par un niveau de tension inférieur, en vue d'empêcher le dispositif de stockage de charge (416) de se décharger sur la ligne de bus.

2. Montage de circuits de commande (518) selon l'une quelconque des revendications précédentes, configuré afin d'être exploitable de manière à déconnecter la ligne de bus du dispositif de stockage de charge (416), en réponse à la transmission, par le montage de circuits de dispositifs périphériques, de la valeur de données représentée par le niveau de tension inférieur.

3. Montage de circuits de commande (518) selon l'une quelconque des revendications précédentes, comprenant un commutateur pour mettre en oeuvre la connexion et la déconnexion du dispositif de stockage de charge (416) et de la ligne de bus.

4. Montage de circuits de commande (518) selon la revendication 3, dans lequel le commutateur est un transistor à effet de champ à semiconducteur à oxyde métallique à mode d'enrichissement à canal P (619).

5. Montage de circuits de commande (518) selon l'une quelconque des revendications précédentes, dans lequel le dispositif de stockage de charge (416) est un condensateur.

6. Circuit intégré à application spécifique (ASIC) (413) comprenant le montage de circuits de commande (518) selon l'une quelconque des revendications précédentes.

7. Montage de dispositifs périphériques, comprenant :
le montage de circuits de commande (518) selon l'une quelconque des revendications 1 à 5 ; ou
le circuit intégré à application spécifique (ASIC) (413) selon la revendication 6.

8. Dispositif périphérique comprenant l'un des éléments ci-dessous :
le montage de circuits de dispositifs périphériques selon la revendication 7 ;
le montage de circuits de commande (518) selon l'une quelconque des revendications 1 à 5 ; ou
le circuit intégré à application spécifique (ASIC) (413) selon la revendication 6.

9. Système comprenant :
au moins un montage de circuits de dispositifs terminaux ; et
un des éléments parmi le montage de circuits de dispositifs périphériques selon la revendication 7 ou le dispositif périphérique selon la revendication 8.

10. Procédé comprenant,
la connexion :
d'une ligne de bus de montage de circuits de dispositifs périphériques, la ligne de bus étant agencée de manière à être apte à transférer des données et de l'énergie entre le montage de circuits de dispositifs périphériques et le montage de circuits de dispositifs terminaux connectables ; à
un dispositif de stockage de charge (416) du montage de circuits de dispositifs périphériques, le dispositif de stockage de charge (416) étant agencé de manière à être apte à recevoir de l'énergie provenant du montage de circuits de dispositifs terminaux sur la ligne de bus, et à fournir de l'énergie au montage de circuits de commande (518), en réponse à la transmission, par le montage de circuits de dispositifs terminaux, d'une première valeur de données représentée par un niveau de tension supérieur, en vue de permettre au dispositif de stockage de charge (416) de recevoir de l'énergie pour un stockage ; et
la déconnexion de la ligne de bus du dispositif de stockage de charge (416) en réponse à la transmission, par le montage de circuits de dispositifs terminaux, d'une seconde valeur de données représentée par un niveau de tension inférieur, en vue d'empêcher le dispositif de stockage de charge (416) de se décharger sur la ligne de bus.

11. Procédé selon la revendication 10, comprenant la déconnexion du dispositif de stockage de charge (416) de la ligne de bus en réponse à la transmission, par le montage de circuits de dispositifs périphériques, de la valeur de données représentée par le niveau de tension inférieur.

12. Produit-programme informatique comprenant un code de programme stocké dans un support lisible par ordinateur, le code de programme informatique étant configuré de manière à mettre en oeuvre l'une quelconque des étapes selon l'une quelconque des revendications 10 et 11, lorsque le programme est exécuté sur un processeur.
